# EUROPEAN PATENT APPLICATION

(11) **EP 4 559 439 A1**
(43) Date of publication of application: **28.05.2025**
(21) Application number: 23841994.9
(22) Date of filing: 20.06.2023
(51) Int. Cl.: A61F 2/90, A61F 2/91, A61B 17/12

(54) **INTRAVASCULAR STENT AND PREPARATION METHOD THEREFOR**

(30) Priority: 20.07.2022 CN 202210858400
(71) Applicant: Conlife Medical Scientific (Shenzhen) Co., Ltd, Shenzhen, Guangdong 518109 (CN)
(72) Inventor: LIU, Xiangdong, Shenzhen, Guangdong 518109 (CN)
(74) Representative: Zaboliene, Reda
(86) International application number: PCT/CN2023/101254
(87) International publication number: WO 2024/016928

(57) **Abstract**

The present application relates to an intravascular stent and a preparation method therefore. The intravascular stent includes: a stent body, developing fasteners, and developing members. The developing fasteners are arranged at the proximal and distal ends of the stent body, and each developing member is fixedly connected to a corresponding developing fastener in a one-to-one manner. Each developing member is fixed on the side of the corresponding developing fastener that is closer to the central axis of the stent body. In the intravascular stent described above, since the developing member is fixed on the side of the developing fastener closer to the central axis of the stent body, it can prevent the developing member from scraping against the inner wall of the microcatheter during delivery, thereby reducing the pushing force required for the intravascular stent and lowering clinical risks. Furthermore, once the intravascular stent is released, it can also prevent the developing member from irritating the inner wall of the blood vessel, thus reducing the risk of thrombosis and neointimal hyperplasia.

## Description

### FIELD

The present invention relates to the technical field of medical instruments, and more particularly, to an intravascular stent and a preparation method therefore.

### BACKGROUND

The portion here is merely background information provided for better understanding of the present disclosure, not necessarily prior arts.

An intracranial aneurysm refers to an abnormal protrusion occurring at the walls of intracranial arteries, which is a primary cause of subarachnoid hemorrhage. After an aneurysm ruptures and bleeds, it not only causes damages to brain tissue but also induces cerebral vasospasm, and the above symptoms typically occur 3 to 14 days after subarachnoid hemorrhage. Blood clots will stimulate the blood vessel walls, causing severe vasoconstriction which can lead to ischemic necrosis of brain tissue, coma, or hemiplegia. Intracranial aneurysms may result from congenital arterial dysplasia, infection, arteriosclerosis, or craniocerebral trauma. Clinically, intracranial aneurysms present as headaches and nausea, and needs to be treated surgically. At present, interventional therapy and surgical craniotomy clipping are the two main surgical methods for intracranial aneurysms. Interventional therapy involves deploying special alloy-based metallic coils into the aneurysm to induce thrombosis and achieve aneurysm embolization, thus reducing the impact of blood flow on the aneurysm wall and achieving hemostasis. However, the stability of these coils is relatively low, limiting their surgical outcome.

Stent-assisted coil embolization is an important technique in the interventional embolization of intracranial aneurysms. With the assistance of stent, it is possible to achieve favorable treatment effects for wide-necked, very small, or fusiform intracranial aneurysms that were previously not amenable to dense embolization or even any embolization, thereby increasing the embolization ratio, preventing aneurysm recurrence, and promoting healing.

The stent mainly serves the following functions:
1. The stent protects the parent artery, such that the coil can be well packed into the aneurysm with the stent serving as a scaffold, preventing post-operative cerebral infarction caused by stenosis or occlusion of the parent artery. 2. The stent increases the neck embolization density, and blocks and supports coils at the aneurysm neck, allowing the coil to fully cover the aneurysm neck, thereby promoting thrombosis and healing. 3. The stent changes the morphology of the parent artery, guides blood flow and reduces blood entering the aneurysm, thus promoting healing. 4. The stent stimulates neointimal growth on the blood vessel, promoting aneurysm healing.

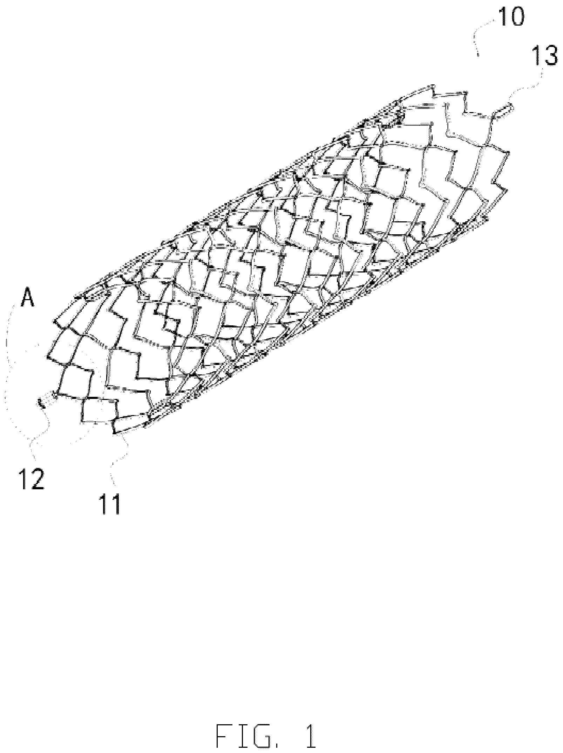

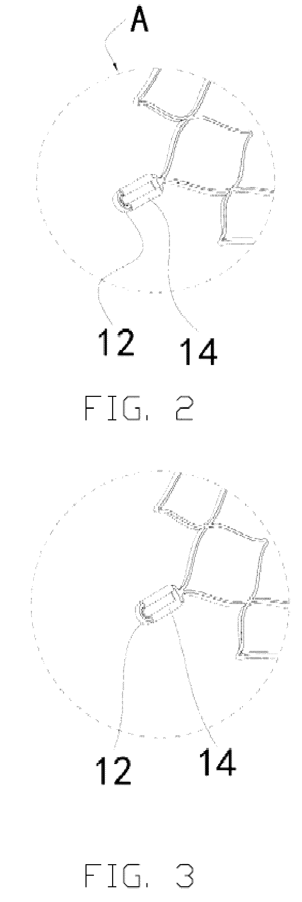

As shown in FIGS. 1 and 2, an intracranial assisting stent 10 in related arts includes a stent body 11, a proximal developing fastener 12, and a distal developing fastener 13. The proximal developing fasteners 12 and the distal developing fasteners 13 are respectively located at the proximal and distal ends of the stent body and are each provided in triplicate for fixing developing materials. The stent body 11 provides the supporting force of the stent.

The end developing structure typically consists of a developing material and a developing fastener. Various configurations of developing structures have been used in the related arts, for example:

As shown in FIG. 2, a developing collar 14 is press-fitted onto the proximal developing fastener 12, with a recessed portion engaged inside the proximal developing fastener 12, thereby forming a proximal developing structure. The distal developing structure is formed similarly.

As shown in FIG. 3, a developing collar 14 is fixed onto the rod of the proximal developing fastener 12, forming a proximal developing structure. The distal developing structure is formed similarly.

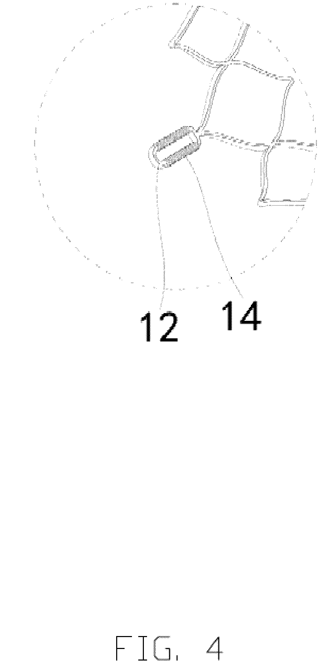

As shown in FIG. 4, a developing wire 14 is wound around the rod of the proximal developing fastener 12, forming a proximal developing structure. The distal developing structure is formed similarly.

The related arts also have problems and disadvantages:
1. In the above three fixation methods for developing materials, a portion of the developing material always resides outside the stent. During delivery, the external developing material may scrape against the inner wall of the microcatheter, causing damage and debris formation, which increases the pushing force needed for the stent and thus poses clinical risks. Also, the external developing material can irritate the inner wall of the blood vessel, causing thrombosis and neointimal hyperplasia.
2. In the above three fixation methods, a gap forms between the developing fastener and the developing material, resulting in severe crevice corrosion in the human blood environment. Crevice corrosion not only leads to corrosion of the metal material but also generates microcurrents that hinder endothelial cells from covering the developing material, thereby turning it into a source of thrombosis.
3. In the above three fixation methods, heavy metals such as gold or platinum are commonly used as developing materials. Although these materials are soft and easy to fix, their electrode potentials are much higher than that of nickel-titanium materials used in the stent (for example, the standard electrode potential of gold is 1.692 V, platinum is 1.18 V, and nickel-titanium alloy is -0.5 V), leading to severe galvanic corrosion in the human blood environment. Galvanic corrosion not only causes material degradation but also produces microcurrents that prevent endothelial cells from adhering to the developing material, again making it a source of thrombosis.
4. Since intracranial stents have a very small diameter, the developing fasteners are extremely narrow and thin (for example, the width of the proximal developing fastener shown in FIG. 3 is typically 0.2-0.3 mm, and the thickness is 0.05-0.06 mm). Press-fitting a developing collar onto such a thin marker fixing member requires very high precision, making it difficult for operation and lowering the production efficiency.

### SUMMARY

The purpose of the present invention is to at least solve one of the above-mentioned problems. The purpose is achieved by the following technical solutions:

An embodiment of the present application provides an intravascular stent including: a stent body, developing fasteners arranged at the proximal and distal ends of the stent body, and developing members fixedly connected to the developing fasteners in a one-to-one correspondence, wherein each developing member is fixed on the side of the corresponding developing fastener closer to the central axis of the stent body.

In one embodiment, both the developing members and the developing fasteners are plate-like structures.

In one embodiment, an edge of the developing member and/or the developing fastener is provided with a first filling layer, which is used to fill the gap between the edges of the developing member and the developing fastener.

In one embodiment, the first filling layer is a first weld seam formed by welding, and the developing member and the developing fastener are fixedly connected via the first weld seam.

In one embodiment, the developing fastener is provided with a first positioning hole, and the developing member is provided with a second positioning hole at a corresponding position, such that the developing member and the developing fastener are aligned via the first positioning hole and the second positioning hole.

In one embodiment, the intravascular stent further includes a positioning pin inserted into the first and second positioning holes, and the positioning pin is fixedly connected to the developing member and the developing fastener.

In one embodiment, an edge of the developing member and/or the developing fastener is provided with a first filling layer used to fill the gap between the edges of the developing member and the developing fastener, and the first filling layer is a parylene coating.

In one embodiment, a second filling layer is provided on the hole walls of the first and second positioning holes, and the second filling layer fills the gap formed at the position of the first and second positioning holes between the developing fastener and the developing member.

In one embodiment, the second filling layer is a second weld seam formed by welding.

In one embodiment, the shape and size of the developing member are identical to those of the developing fastener.

In one embodiment, the difference in electrode potential between the developing fastener and the developing member is less than 1 V.

In one embodiment, the developing fastener is made of a nickel-titanium alloy, and the developing member is made of tantalum.

In one embodiment, a parylene coating is provided on the stent body.

Through the above configuration, since the developing member is fixed on the side of the developing fastener closer to the central axis of the stent body, the developing member is arranged on the inner side of the developing fastener. During delivery, the developing member does not contact the microcatheter, preventing the element from scraping the inner wall of the microcatheter, reducing the pushing force of the intravascular stent and lowering clinical risks. After the intravascular stent is released, the developing member does not directly contact the vessel wall, avoiding irritation to the vessel wall and reducing the risk of thrombosis and neointimal hyperplasia.

Furthermore, by providing the first filling layer, the gap between the developing fastener and the developing member can be filled, preventing crevice corrosion in the human blood environment and slowing down metal corrosion. This also reduces the microcurrent caused by crevice corrosion, accelerating endothelial cell coverage of the developing member and improving the biocompatibility of the intravascular stent.

In addition, since the difference in electrode potential between the developing member and the developing fastener is small, metal corrosion is slowed, and galvanic corrosion currents are reduced, facilitating endothelial cell coverage and improving the stent's biocompatibility.

Moreover, by using a positioning pin and the first and second positioning holes, the developing member and developing fastener can be accurately aligned. Subsequently, the developing member is fixed onto the developing fastener by welding, resulting in lower precision requirements, reduced difficulty, and higher production efficiency.

An embodiment of the present application also provides a preparation method for an intravascular stent, including:
Step S1: Providing a stent body with developing fasteners at its proximal and distal ends;
Step S2: Providing developing members in a quantity matching that of the developing fasteners;
Step S3: Fixing the developing members on the sides of the developing fasteners closer to the central axis of the stent body.

In one embodiment, both the developing members and the developing fasteners are plate-like structures; the developing fastener is provided with a first positioning hole, and the developing member is provided with a corresponding second positioning hole. In step S3, the method includes: passing a positioning pin respectively through the first and second positioning holes, placing the developing member on the side of the developing fastener closer to the central axis of the stent body, and then fixing the developing member onto the developing fastener.

In one embodiment, the step of fixing the developing member onto the developing fastener specifically includes: forming a first weld seam at the edges of the developing member and the developing fastener by laser welding, and fixing the developing member onto the developing fastener via the first weld seam.

In one embodiment, step S3 further includes: after aligning the developing member and the developing fastener, removing the positioning pin, and welding at the contact position of the first and second positioning holes to form a second weld seam that fills the gap at the position of these holes.

In one embodiment, step S3 includes: forming welding pools at both ends of the positioning pin by welding, thereby fixing the positioning pin, the developing member, and the developing fastener together.

In one embodiment, after step S3, a parylene coating is applied onto the developing member and the developing fastener.

In one embodiment, after step S3, a parylene coating is applied onto the stent body.

The preparation method of the intravascular stent described above requires lower precision, reduced difficulty, and higher production efficiency.

Moreover, the intravascular stent manufactured by the above method ensures that, during delivery, the developing member does not contact the microcatheter, preventing scraping against the inner wall of the microcatheter and reducing pushing force and clinical risks. After the stent is released, the developing member does not directly contact the vessel wall, avoiding irritation and reducing thrombosis and neointimal hyperplasia.

### BRIEF DESCRIPTION OF THE DRAWINGS

Various other advantages and benefits will become clearer to those skilled in the art from the detailed description of the following preferred embodiments. The drawings are provided merely for illustrating the preferred embodiments and are not to be considered limiting. Identical reference signs refer to identical parts throughout the drawings.
FIG. 1 is a diagrammatic view of an intravascular stent according to the prior art.
FIG. 2 is an enlarged view of region A in FIG. 1.
FIG. 3 is a diagrammatic view of another developing structure according to the prior art.
FIG. 4 is a diagrammatic view of yet another developing structure according to the prior art.
FIG. 5 is a diagrammatic view of an intravascular stent according to an embodiment of the present application.
FIGS. 6 to 8 are diagrammatic views illustrating the assembly process of the developing member and the developing fastener of the intravascular stent shown in FIG. 5.
FIG. 9 is a partial diagrammatic view of an intravascular stent according to a second embodiment of the present application.
FIG. 10 is a partial diagrammatic view of an intravascular stent according to a third embodiment of the present application.
FIG. 11 is a sectional view along line B-B in FIG. 10.

### DETAILED DESCRIPTION

Below, exemplary embodiments of the present disclosure will be described in more detail with reference to the accompanying drawings. Although the accompanying drawings show exemplary embodiments of the present disclosure, it should be understood that the present disclosure can be implemented in various forms without being limited to the embodiments described herein. Instead, these embodiments are provided so that the present disclosure can be thoroughly understood, and the full scope of the present disclosure can be conveyed to those skilled in the art.

It should be understood that the terms used herein are merely for describing particular exemplary embodiments and are not intended to limit the invention. Unless otherwise explicitly indicated, the singular forms "a," "an," and "the" as used herein may also include plural forms. The terms "comprise," "comprising," "include," "including," and "have," when used herein, are inclusive and thus indicate the presence of the stated features, steps, operations, elements, and/or components, but do not preclude the presence or addition of one or more other features, steps, operations, elements, components, and/or combinations thereof.

Unless clearly indicated otherwise from the context, the terms such as "first," "second," and "third" used herein to describe multiple elements, components, regions, layers, and/or sections do not imply any sequence or order. These terms are merely used to distinguish one element, component, region, layer, or section from another. Therefore, a first element, component, region, layer, or section discussed below may also be referred to as a second element, component, region, layer, or section without departing from the teachings of the exemplary embodiments. Thus, numerical terms such as "first" and "second" do not imply any sequence or order unless explicitly stated.

For convenience, spatial relative terms may be used herein to describe the relationship of one element or feature with respect to another element or feature as shown in the figures. Such relative spatial terms, for example "inner," "outer," "inside," "outside," "below," "beneath," "above," "over," etc., are intended to encompass different orientations of the device in use or operation in addition to the orientation depicted in the figures. For example, if the device in the figures is flipped over, elements or features described as "below" or "beneath" other elements or features would then be oriented "above," and vice versa. Thus, exemplary terms such as "below" may include both orientations above and below. The device may be otherwise oriented (rotated by 90 degrees or at other angles) and the relative spatial descriptors used herein are to be interpreted accordingly.

Unless otherwise defined, all technical and scientific terms used herein have the meaning commonly understood by those skilled in the art to which this invention belongs. The terminology used in the description herein of the present invention is only for describing particular embodiments, and is not intended to limit the present invention. The term "and/or" as used herein includes any and all combinations of one or more of the associated listed items.

It should be noted that the ranges expressed from "a numerical value to another numerical value" in the present application represent a concise method of expression to avoid enumerating all values in that range one by one in the specification. Therefore, the recitation of a specific numerical value range covers any numerical value within that range, as well as smaller ranges formed by any two values within that range, as if such values and smaller ranges were explicitly stated in the specification.

It should be noted that in the present application, the end closer to the operator is referred to as the "proximal end," and the end farther from the operator is referred to as the "distal end." Based on this principle, the "proximal end" and "distal end" of any component of the intravascular stent can be defined.

The present application provides an intravascular stent that can be used for intracranial aneurysm vascular treatment, such as being used as an assisting stent in stent-assisted coil embolization procedures. The intravascular stent includes a stent body, developing fasteners arranged at the proximal and distal ends of the stent body, and developing members fixedly connected to the developing fasteners.

The stent body is formed by laser cutting a metal tube (such as a Ni-Ti tube), and the tube wall of the metal tube forms multiple hollowed-out meshes. The developing fasteners and the stent body are integrally formed by laser cutting. In one embodiment, three developing fasteners are provided at both the proximal and distal ends of the stent body.

Both the developing fasteners and the developing members are plate-like structures. The developing member is fixed on the side of the developing fastener closer to the central axis of the stent body. That is, the developing member is disposed on the inner surface of the developing fastener. During delivery, the developing member does not contact the inner wall of the microcatheter, thereby avoiding scraping the microcatheter's inner wall, reducing the pushing force of the intravascular stent, and lowering clinical risks. Moreover, after the intravascular stent is released, the developing member also does not come into direct contact with the vessel wall, avoiding irritation to the vessel wall and reducing the risks of thrombosis and neointimal hyperplasia.

In one embodiment, an edge of the developing member and/or the developing fastener is provided with a first filling layer. The first filling layer is used to fill the gap between the edges of the developing member and the developing fastener. For example, the first filling layer may be a first weld seam formed by welding, and the developing member and the developing fastener are fixedly connected via the first weld seam. Alternatively, the first filling layer may be a parylene coating. By providing the first filling layer, gaps between the developing fastener and the developing member can be filled, thereby preventing the formation of severe crevice corrosion in the human blood environment, delaying metal corrosion, and simultaneously reducing the microcurrent caused by crevice corrosion. This can accelerate the endothelial coverage on the developing member and improve the biocompatibility of the intravascular stent.

In one embodiment, the developing fastener is provided with a first positioning hole, and the developing member is provided with a second positioning hole at a corresponding position. The developing member and the developing fastener are aligned by means of the first positioning hole and the second positioning hole. By providing the first positioning hole and the second positioning hole, alignment of the developing member with the developing fastener is facilitated. In one embodiment, a positioning pin is used to pass through the first positioning hole and the second positioning hole for achieving precise alignment of the developing member and the developing fastener. In one embodiment, both the first positioning hole and the second positioning hole are two in number, arranged in the middle positions of the developing fastener and the developing member.

In one embodiment, a second filling layer is provided on the hole walls of the first positioning hole and the second positioning hole. The second filling layer is used to fill the gap at the position of the first positioning hole and the second positioning hole between the developing fastener and the developing member. In one embodiment, the second filling layer is a second weld seam formed by welding. In one embodiment, after aligning the developing member and the developing fastener, the positioning pin is removed, and the position where the first positioning hole and the second positioning hole contact is welded to form the second weld seam, thereby filling the gap at that position. In another embodiment, the positioning pin may be retained, and welding is performed at both ends of the positioning pin to form welding pools, not only eliminating gaps but also improving the shear strength between the developing member and the developing fastener, reducing the risk of the developing member detaching from the developing fastener. Moreover, the edge of the developing member and/or the developing fastener is provided with the first filling layer, which can be a parylene coating. In one embodiment, parylene coatings are provided on the developing member, the developing fastener, and the stent body.

In one embodiment, the shape and size of the developing member are identical to those of the developing fastener. Of course, in other embodiments, the shape and size of the developing member do not necessarily have to be completely identical to those of the developing fastener. For example, the developing member can be slightly smaller than the developing fastener.

In one embodiment, the difference in electrode potential between the developing fastener and the developing member is less than 1 V. In one embodiment, the developing member is made of tantalum, and the developing fastener is made of a nickel-titanium alloy. The standard electrode potential of tantalum is -0.750 V, which is much lower than that of gold and platinum, and closer to the standard electrode potential of the nickel-titanium alloy. The galvanic corrosion potential difference formed between tantalum and the nickel-titanium alloy is 0.25 V, whereas the potential differences formed by gold and platinum with nickel-titanium alloy are 2.192 V and 1.68 V, respectively. In other words, using tantalum as the developing member material, compared with gold and platinum, reduces the galvanic corrosion potential difference with nickel-titanium alloy by about 90% and 95%, respectively. This can delay metal corrosion and reduce galvanic corrosion currents, facilitating endothelial cell coverage on the metal materials and improving the biocompatibility of the intravascular stent.

The present application further provides a preparation method for an intravascular stent, including the following steps:
Step S1: Providing a stent body with developing fasteners, the developing fasteners being arranged at the proximal and distal ends of the stent body;
Step S2: Providing developing members, the quantity of the developing members being the same as that of the developing fasteners;
Step S3: Fixing each developing member onto the side of the corresponding developing fastener that is closer to the central axis of the stent body. For example, the developing member can be fixed onto the side of the developing fastener closer to the central axis of the stent body by welding.

In one embodiment, both the developing fasteners and the developing members are plate-like structures. The developing fastener is provided with a first positioning hole, and the developing member is provided with a second positioning hole at the corresponding position. During Step S3, the process specifically comprises: passing a positioning pin through the first positioning hole, then threading the second positioning hole onto the positioning pin so that the developing member is positioned on the side of the developing fastener closer to the central axis of the stent body, and then fixing the developing member onto the developing fastener. In one embodiment, the step of fixing the developing member onto the developing fastener includes forming a first weld seam by laser welding at the edges of the developing member and the developing fastener, thereby fixing the developing member onto the developing fastener through the first weld seam.

In one embodiment, Step S3 further comprises: after aligning the developing member with the developing fastener, removing the positioning pin, and then welding the contact position of the first positioning hole and the second positioning hole between the developing member and the developing fastener to form a second weld seam, so as to fill the gap at the position of the first and second positioning holes between the developing member and the developing fastener. In another embodiment, Step S3 comprises: forming welding pools at both ends of the positioning pin by welding, so that the positioning pin, the developing member, and the developing fastener are all fixed together. In this embodiment, the material of the positioning pin is the same as that of the developing fastener.

In one embodiment, after Step S3, a parylene coating is applied on the developing member and the developing fastener. Specifically, after assembling the developing member and the developing fastener, a parylene coating is then applied or deposited. In one embodiment, after Step S3, a parylene coating is further applied on the stent body.

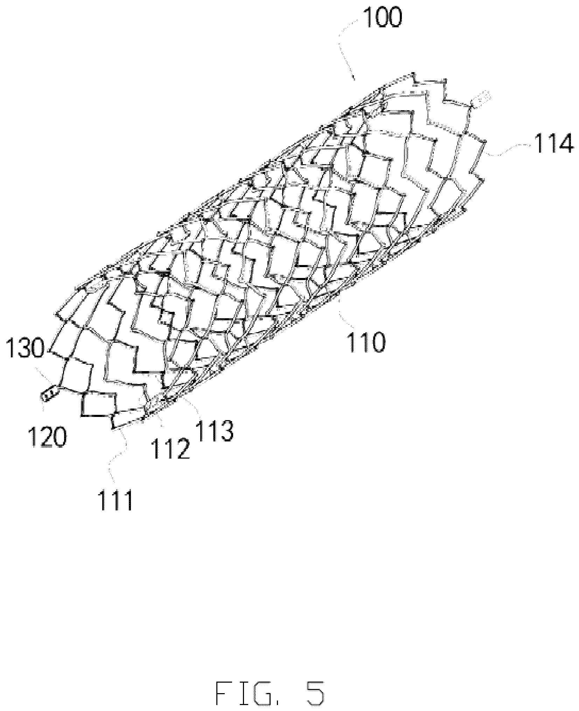

Referring to FIG. 5. An embodiment of the intravascular stent 100 of the present application includes a stent body 110, developing fasteners 120, and developing members 130. The developing fasteners 120 are arranged at the proximal and distal ends of the stent body 110. The stent body 110 is formed by laser cutting a metal tube (e.g., Ni-Ti tube), and the tube wall of the metal tube forms multiple hollowed-out meshes. In the illustrated embodiment, the stent body 110 includes a row of proximal closed-ring wave loops 111, multiple rows of open-ring coarse wave loops 112, multiple rows of open-ring fine wave loops 113, and a distal supporting wave loop 114. The proximal closed-ring wave loop 111 is located at the proximal end of the stent body 110, the multiple rows of open-ring coarse wave loops 112 and fine wave loops 113 are located in the middle region of the stent body 110, with the coarse wave loops 112 and fine wave loops 113 alternating. The distal supporting wave loop 114 is located at the distal end of the stent body 110. The developing fasteners 120 and the stent body 110 are integrally formed by laser cutting. In one embodiment, both the proximal and distal ends of the stent body 110 are each provided with three developing fasteners 120, evenly distributed along the circumferential direction of the stent body 110.

It should be noted that the stent body 110 can have other structures. The present application does not limit the structure of the stent body. The number of developing fasteners 120 can also be selected as needed. The numbers of developing fasteners 120 at the proximal and distal ends may be the same or different.

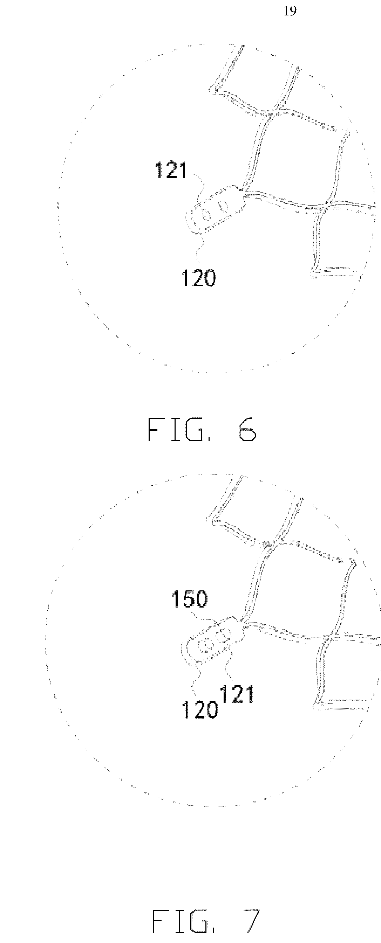

Referring to FIGS. 6 to 8. The developing fastener 120 is a plate-like structure, and the developing member 130 is also a plate-like structure. The developing member 130 is fixed on the side of the developing fastener 120 closer to the central axis of the stent body 110, that is, the developing member 130 is disposed on the inner side of the developing fastener 120. During delivery, the developing member 130 does not contact the inner wall of the microcatheter, thus avoiding scraping the inner wall of the microcatheter, reducing the pushing force of the intravascular stent 100 and minimizing clinical risk. After the intravascular stent 100 is released, the developing member 130 also does not directly contact the vessel wall, preventing the developing member from irritating the vessel wall and reducing the risk of thrombosis and neointimal hyperplasia. In one embodiment, the developing member 130 is fixed to the developing fastener 120 by welding.

Referring to FIGS. 6 to 8. The developing fastener 120 is provided with the first positioning hole 121, and the developing member 130 is provided with the second positioning hole 131. The developing member 130 and the developing fastener 120 are aligned by means of the first positioning hole 121 and the second positioning hole 131. The shape and size of the developing member 130 are identical to those of the developing fastener 120. In actual operation, after the positioning pin 150 is passed through the first positioning hole 121, the second positioning hole 131 is then aligned with the positioning pin 150, achieving precise alignment of the developing member 130 and the developing fastener 120.

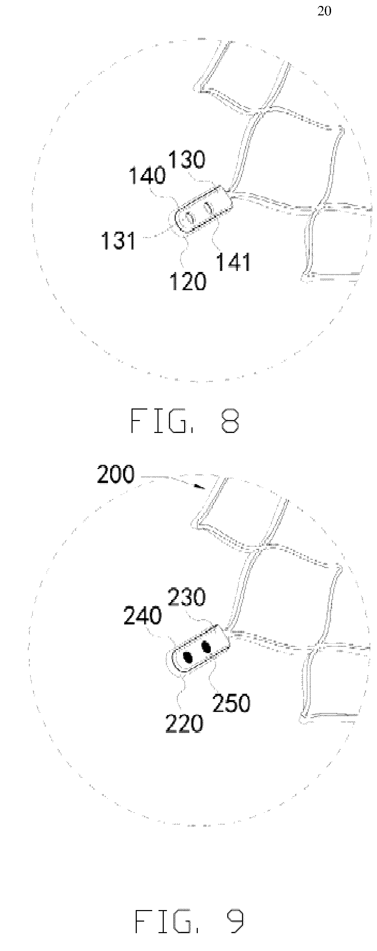

Referring to FIG. 8. A first weld seam 140 is formed by welding the edges of the developing member 130 and the developing fastener 120, thereby filling the gap between them. This prevents crevice corrosion in the human blood environment, delays metal corrosion, and reduces the microcurrent of crevice corrosion, accelerating endothelial coverage on the developing member and improving the stent's biocompatibility. In the illustrated embodiment, after welding the outer edges of the developing member 130 and the developing fastener 120, the positioning pin 150 is removed. Then, at the contact position of the first positioning hole 121 and the second positioning hole 131, a second weld seam 141 is formed by welding, further preventing crevice corrosion. With the aid of the positioning pin 150 for precise alignment of the first positioning hole 121 and the second positioning hole 131, and then using a welding process to fix the developing member, the process requires lower precision, is less difficult, and has higher production efficiency.

In the illustrated embodiment, each developing fastener 120 is provided with two first positioning holes 121, and correspondingly, each developing member 130 is provided with two second positioning holes 131.

In one embodiment, the developing member 130 is made of tantalum, and the developing fastener 120 is made of a nickel-titanium alloy. Tantalum has a standard electrode potential of -0.750 V. Compared with gold and platinum's standard electrode potentials, tantalum's electrode potential is significantly lower and closer to that of nickel-titanium alloy. The galvanic corrosion potential difference formed between tantalum and nickel-titanium alloy is 0.25 V, whereas using gold or platinum as the developing member results in galvanic corrosion potential differences of 2.192 V and 1.68 V, respectively. Thus, using tantalum as the developing member material can reduce the galvanic corrosion potential difference by approximately 90% and 95%, compared with gold and platinum, respectively. This slows metal corrosion and reduces galvanic corrosion currents, thereby promoting endothelial cell coverage on the metal materials and improving the biocompatibility of the intravascular stent 100.

In one embodiment, the width of the developing member 130 and the developing fastener 120 is both 0.2 mm-0.3 mm, and the thickness is 0.05 mm-0.06 mm.

Referring to FIG. 9. A second embodiment of the intravascular stent 200 of the present application is generally similar in structure to the intravascular stent 100 of the first embodiment. The main difference is that the intravascular stent 200 includes a positioning pin 250. The positioning pin 250 is inserted into the first positioning hole (blocked by the positioning pin 250) and the second positioning hole (blocked by the positioning pin 250), and the positioning pin 250 is fixedly connected with the developing member 230 and the developing fastener 220. In one embodiment, after passing the positioning pin 250 through the first and second positioning holes, laser welding is performed at both ends of the positioning pin 250 to fix both ends of the positioning pin 250 to the developing member 230 and the developing fastener 220. In this embodiment, the positioning pin 250 may be a Ni-Ti wire.

With the intravascular stent 200, due to the presence of the positioning pin 250, the shear strength between the developing fastener 220 and the developing member 230 is enhanced, preventing the developing member 230 from detaching from the developing fastener 220.

In one embodiment, the edges of the developing member 230 and the developing fastener 220 are welded by laser to form a first weld seam 240, which can eliminate gaps between the developing member 230 and the developing fastener 220, thereby avoiding crevice corrosion caused by gaps after the intravascular stent 200 is implanted.

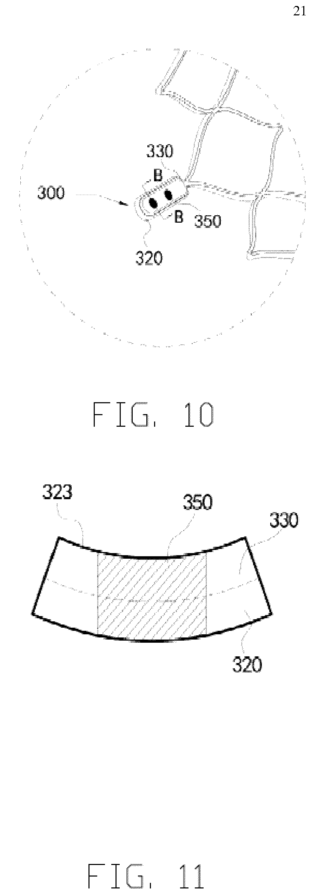

Referring to FIGS. 10 and 11. A third embodiment of the intravascular stent 300 of the present application is generally similar in structure to the intravascular stent 200 of the second embodiment. The main difference is that for the intravascular stent 300, the edges of the developing member 330 and the developing fastener 320 are not welded to form a first weld seam. Instead, a parylene coating 343 is provided on the developing fastener 320 and the developing member 330. The parylene coating can fill the gap between the developing member 330 and the developing fastener 320. Moreover, parylene coating 343 has good electrical insulation, preventing crevice corrosion between the developing member 330 and the developing fastener 320. In one embodiment, after assembling the developing member 330 and the developing fastener 320 via the positioning pin 350, a parylene coating 343 is then applied. In one embodiment, the thickness of the parylene coating 343 is 0.1-1 micron.

In one embodiment, a parylene coating is also applied to the stent body. When the intravascular stent 300 is used to assist coil embolization of an aneurysm, due to the good electrical insulation of parylene, the parylene coating can isolate the contact between the intravascular stent and the metal coil, thereby preventing galvanic corrosion currents. This protects the intravascular stent and improves the biocompatibility by accelerating reendothelialization, reducing the risk of thrombosis after implanting the intravascular stent 300.

In the above embodiments, various technical features can be arbitrarily combined. For brevity, all possible combinations of technical features in the above embodiments are not exhaustively described. Nevertheless, as long as such combinations do not result in contradictions, they should be considered within the scope described in this specification.

The above-mentioned embodiments are only illustrative examples of the present invention's implementation, and are described in detail. However, these should not be interpreted as limitations on the scope of the invention. It should be understood that, for those skilled in the art, various changes and modifications can be made without departing from the spirit and scope of the invention. Therefore, the protection scope of the present invention shall be defined by the appended claims.

## Claims

1. An intravascular stent, **characterized by** comprising a stent body, developing fasteners, and developing members, wherein the developing fasteners are arranged at a proximal end and a distal end of the stent body, each of the developing members is fixed to a corresponding one of the developing fasteners in a one-to-one manner, and each of the developing members is fixed on a side of the corresponding one of the developing fasteners closer to a central axis of the stent body.

2. The intravascular stent according to claim 1, **characterized in that**, each of the developing members and the developing fasteners are plate-like structures.

3. The intravascular stent according to claim 2, **characterized in that**, an edge of the developing member and/or the developing fastener is provided with a first filling layer, the first filling layer being configured to fill a gap between the edge of the developing member and the edge of the developing fastener.

4. The intravascular stent according to claim 3, **characterized in that**, the first filling layer is a first weld seam formed by welding, and the developing member and the developing fastener are fixed to each other by the first weld seam.

5. The intravascular stent according to claim 2, **characterized in that**, the developing fastener is provided with a first positioning hole, the developing member is provided with a second positioning hole accordingly, wherein the developing member and the developing fastener are aligned with each other through the first positioning hole and the second positioning hole.

6. The intravascular stent according to claim 5, **characterized in that**, the intravascular stent further comprises a positioning pin, the positioning pin is inserted into the first positioning hole and the second positioning hole, and fixed to the developing member and the developing fastener.

7. The intravascular stent according to claim 6, **characterized in that**, an edge of the developing member and/or the developing fastener is provided with a first filling layer, the first filling layer being configured to fill a gap between the edge of the developing member and the edge of the developing fastener, and the first filling layer is a parylene coating.

8. The intravascular stent according to claim 5, **characterized in that**, a second filling layer is provided on a hole wall of each of the first positioning hole and the second positioning hole, and the second filling layer is configured to fill the gap formed at a position of the first positioning hole and the second positioning hole between the developing fastener and the developing member.

9. The intravascular stent according to claim 8, **characterized in that**, the second filling layer is a second weld seam formed by welding.

10. The intravascular stent according to claim 2, **characterized in that**, a shape and size of the developing member are the same as those of the developing fastener.

11. The intravascular stent according to claim 1, **characterized in that**, a difference in electrode potential between the developing fastener and the developing member is less than 1 V.

12. The intravascular stent according to claim 1, **characterized in that**, the developing fastener is made of a nickel-titanium alloy, and the developing member is made of tantalum.

13. The intravascular stent according to any one of claims 1 to 12, **characterized in that**, a parylene coating is provided on the stent body.

14. A preparation method for an intravascular stent, **characterized by** comprising the following steps:
Step S1: providing a stent body with developing fasteners, and the developing fasteners being arranged at a proximal and a distal end of the stent body;
Step S2: providing developing members, a quantity of the developing members being equal to that of the developing fasteners;
Step S3: fixing each of the developing members onto a side of a corresponding one of the developing fasteners closer to a central axis of the stent body.

15. The preparation method according to claim 14, **characterized in that**, each of the developing members and the developing fasteners are plate-like structures, wherein the developing fastener is provided with a first positioning hole, the developing member is provided with a second positioning hole accordingly; in step S3, the method further comprises: passing a positioning pin through the first positioning hole and the second positioning hole, respectively, causing each of the developing members to be positioned at the side of the corresponding one of the developing fasteners closer to the central axis of the stent body, and fixing each of the developing members onto the corresponding one of the developing fasteners.

16. The preparation method according to claim 15, **characterized in that**, the step of fixing the developing member onto the developing fastener specifically comprises: forming a first weld seam at the edges of the developing member and the developing fastener by laser welding, and fixing the developing member onto the developing fastener via the first weld seam.

17. The preparation method according to claim 16, **characterized in that**, step S3 further comprises: after aligning the developing member and the developing fastener, removing the positioning pin, and welding at the contact position of the first positioning hole and the second positioning hole between the developing member and the developing fastener to form a second weld seam, so as to fill the gap formed at the position of the first positioning hole and the second positioning hole between the developing fastener and the developing member.

18. The preparation method according to claim 14 or 15, **characterized in that**, step S3 comprises: forming welding pools at both ends of the positioning pin by welding, thereby fixing the positioning pin, the developing member, and the developing fastener together.

19. The preparation method according to claim 18, **characterized in that**, after step S3, a parylene coating is applied onto the developing member and the developing fastener.

20. The preparation method according to claim 19, **characterized in that**, after step S3, a parylene coating is applied onto the stent body.
